# EUROPEAN PATENT APPLICATION

(11) **EP 4 462 117 A1**
(43) Date of publication of application: **13.11.2024**
(21) Application number: 23873187.1
(22) Date of filing: 27.09.2023
(51) Int. Cl.: G01N 33/44

(54) **SUPER ABSORBENT POLYMER**

(30) Priority: 28.09.2022 KR 20220123440; 26.09.2023 KR 20230129631
(71) Applicant: LG Chem, Ltd., Yeongdeungpo-gu Seoul 07336 (KR)
(72) Inventor: KIM, Tae Yun, Daejeon 34122 (KR); PARK, Se Yeol, Daejeon 34122 (KR)
(74) Representative: Plasseraud IP
(86) International application number: PCT/KR2023/014986
(87) International publication number: WO 2024/072076

(57) **Abstract**

The present disclosure relates to super absorbent polymer that not only has excellent absorption speed, but also has little rewet and leakage when applied to a product such as a diaper, and the like, and has excellent absorption capacity.

## Description

### [Technical Field]

### Cross-Reference to Related Application(s)

This application claims the benefit of Korean Patent Application No. 10-2022-0123440 filed on September 28, 2022 and Korean Patent Application No. 10-2023-0129631 filed on September 26, 2023 with the Korean Intellectual Property Office, the disclosures of which are herein incorporated by reference in their entirety.

The present disclosure relates to super absorbent polymer.

### [Background Art]

Super absorbent polymer (SAP) is synthetic polymer material that can absorb moisture of 500 to 1000 times of self-weight, and is also named differently as super absorbency material (SAM), absorbent gel material (AGM), etc. according to developing companies. The superabsorbent polymer began to be commercialized as sanitary items, and currently, it is being widely used as water-holding material for soil, water stop material for civil engineering and architecture, sheets for raising seedling, freshness preservatives in the field of food circulation, fomentation material, etc.

Since super absorbent polymer used in hygiene products such as diapers, and the like require rapid absorption speed, study and development for improving the same are being progressed. Commonly, the absorption speed of super absorbent polymer is evaluated by a vortex measuring method wherein a certain amount of super absorbent polymer is put in physiological saline and stirred, and a time taken until vortex disappears is measured. However, even if the vortex absorption speed of super absorbent polymer is excellent, in case absorption is locally achieved or a diffusion area is narrow, if a pressure by the weight of a user is applied when the super absorbent polymer is used in a diaper, and the like, a rewet phenomenon wherein a part of the absorbed liquid exudes again may occur, and to the contrary, in case the diffusion area of super absorbent polymer is too wide, leakage of liquid may occur.

### [Disclosure of Invention]

### [Technical Problem]

Thus, it is an object of the present disclosure to provide super absorbent polymer that not only has excellent absorption speed, but also has little rewet and leakage when applied to a product such as a diaper, and the like, and has improved absorption capacity.

### [Technical Solution]

According to one embodiment of the present disclosure, there is provided super absorbent polymer wherein when the super absorbent polymer is spread on the surface of a porous substrate to which physiological saline (0.9 wt%, NaCl aqueous solution) is continuously fed, and the amount of physiological saline that the super absorbent polymer absorbs from the surface of the porous substrate, and the absorption speed are measured continuously from the time the super absorbent polymer is spread, the maximum value of the absorption speed (Vmax) is greater than 0.4 g/g·sec, a time when the maximum value of the absorption speed (Tmax) is achieved, is greater than 5 seconds to less than 120 seconds, and the amount of the physiological saline absorbed in the super absorbent polymer for 5 minutes (C5min) is greater than 40 g.

According to one embodiment, i) the maximum value of the absorption speed (Vmax) may be 0.45 g/g sec to 1 g/g sec.

According to one embodiment, ii) the time when the maximum value of the absorption speed (Tmax) is achieved, may be 8 seconds to 40 seconds.

According to one embodiment, iii) the amount of physiological saline absorbed in the super absorbent polymer for 5 minutes (C5min) may be 45 g to 55 g.

### [Effects]

The super absorbent polymer of the present disclosure not only has excellent absorption speed, but also has excellent absorption and diffusion performances, and thus, has little rewet and leakage, and excellent absorption capacity. Thus, the super absorbent polymer can be suitably used for absorbent products requiring high absorption performance and feeling of use.

### [Brief Description of Drawings]

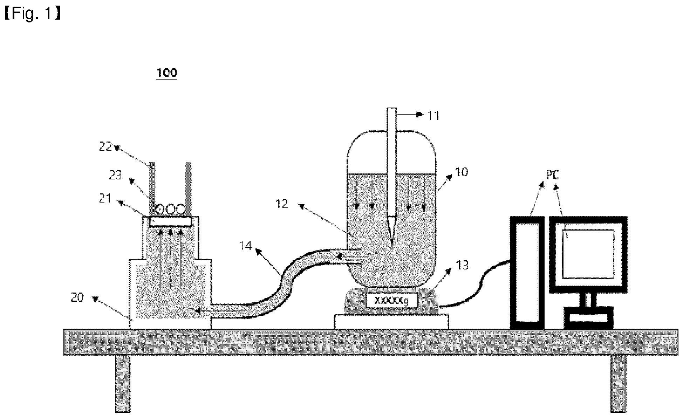

Fig. 1 shows one embodiment of a measuring apparatus that can measure the properties of the super absorbent polymer of the present disclosure.

### [Best Mode for Carrying Out the Invention]

The terms used herein are only to explain specific embodiments, and are not intended to limit the present disclosure. A singular expression includes a plural expression thereof, unless the context clearly indicates otherwise. Throughout the specification, the terms "comprise", "equipped" or "have", etc. are intended to designate the existence of practiced characteristic, number, step, constructional element or combinations thereof, and they are not intended to preclude the possibility of existence or addition of one or more other characteristics, numbers, steps, constructional elements or combinations thereof.

Although various modifications can be made to the present disclosure and the present disclosure may have various forms, specific examples will be illustrated and explained in detail below. However, it should be understood that they are not intended to limit the present disclosure to specific disclosure, and, that the present disclosure includes all the modifications, equivalents or replacements thereof without departing from the spirit and technical scope of the present disclosure.

Technical terms used herein are only for mentioning specific embodiments, and they are not intended to restrict the present disclosure. The singular expressions used herein may include the plural expressions unless the context clearly indicates otherwise.

In the drawings, parts irrelevant to explanations are omitted to clearly explain the present disclosure, and like reference numerals in the specification denote like or similar constructional elements.

### Super absorbent polymer

According to one embodiment of the present disclosure, there is provided super absorbent polymer wherein when the super absorbent polymer is spread on the surface of a porous substrate to which physiological saline (0.9 wt%, NaCl aqueous solution) is continuously fed, and the amount of physiological saline that the super absorbent polymer absorbs from the surface of the porous substrate, and the absorption speed are measured continuously from the time the super absorbent polymer is spread, the maximum value of the absorption speed (Vmax) is greater than 0.4 g/g sec, a time when the maximum value of the absorption speed (Tmax) is achieved, is greater than 5 seconds to less than 120 seconds, and the amount of physiological saline absorbed in the super absorbent polymer for 5 minutes (C5min) is greater than 40 g.

As stated above, super absorbent polymer simultaneously meeting predetermined ranges of Vmax, Tmax, and C5min are excellent in terms of absorption force, absorption speed, and rewet and leakage prevention properties, and thus, may exhibit excellent feeling of use when used in hygiene products such as diapers, and the like.

The Vmax, Tmax, and C5min are values measured at atmospheric pressure (760 ± 50 mmHg) and a temperature of 23°C to 25°C, and the measurement method will be explained in detail later.

The maximum value of absorption speed (Vmax) is related to leakage prevention property of super absorbent polymer. Specifically, when Vmax is greater than 0.4 g/g sec, it may be judged that leakage prevention property of super absorbent polymer is excellent. It is preferable that Vmax is 0.45 g/g sec or more, 0.5 g/g sec or more, and the upper limit is not specifically limited, but for example, it may be 1 g/g sec or less.

The time when the maximum value of absorption speed (Tmax) is achieved, is related to diffusion performance of super absorbent polymer, and specifically, the higher the local absorption power of super absorbent polymer, the shorter the Tmax appears. If the local absorption power of super absorbent polymer is too high, the area in which liquid diffuses in the super absorbent polymer may become narrow, and thus, when the super absorbent polymer is included in a diaper, a rewet phenomenon wherein liquid retained in the super absorbent polymer exudes again by pressurization may occur. To the contrary, if Tmax is too long, leakage may occur. Thus, it is preferable that Tmax is greater than 5 seconds to less than 120 seconds, and more preferably, it may be 6 seconds or more, or 8 seconds or more, or 10 seconds or more, and 60 seconds or less, or 40 seconds or less, or 20 seconds or less.

The amount of physiological saline absorbed in the super absorbent polymer for 5 minutes (C5min) is related to the absorption capacity of super absorbent polymer, and the higher the absorption capacity of super absorbent polymer, the higher the C5min appears. If C5min is less than 40 g, the absorption capacity of super absorbent polymer may not be sufficient, and thus, when applied for hygiene products such as diapers, and the like, the content of super absorbent polymer should be increased, and it may be impossible to produce a thin product, and thus, it is preferable that C5min is greater than 40 g, and it is more preferable that C5min is 42 g or more, or 45 g or more. Theoretically, the upper limit of C5min is not limited, but for example, it may be 60 g or less, or 55 or less.

The super absorbent polymer according to one embodiment of the present disclosure may have the maximum value of absorption speed (Vmax) of 0.45 g/g sec to 1 g/g sec, a time when the maximum value of the absorption speed (Tmax) is achieved, of 8 to 40 seconds, and the amount of the physiological saline absorbed in the super absorbent polymer for 5 minutes (C5min) of 42 g to 55 g.

The super absorbent polymer according to one embodiment of the present disclosure may have the maximum value of absorption speed (Vmax) of 0.5 g/g sec to 1 g/g sec, a time when the maximum value of the absorption speed (Tmax) is achieved, of 10 to 20 seconds, and the amount of the physiological saline absorbed in the super absorbent polymer for 5 minutes (C5min) of 45 g to 55 g.

The Vmax, Tmax, and C5min of super absorbent polymer may be confirmed by measuring the amount of saline that the super absorbent polymer absorbs from the surface of the porous substrate to which physiological saline (0.9 wt%, NaCl aqueous solution) is continuously fed, with the passage of time.

Since the existing property measurement of super absorbent polymer is conducted while making liquid absorbed on the entire surface of super absorbent polymer particles, there is a limitation on the expectation of the actual absorption pattern of super absorbent polymer, and it was difficult to confirm the absorption area or diffusion performance of super absorbent polymer. To the contrary, since the Vmax, Tmax, and C5min are values measured presuming that liquid is absorbed on one surface of super absorbent polymer just like when super absorbent polymer is included in an absorbent product such as a diaper, and the like, all the properties when super absorbent polymer is applied to a product can be more reliably estimated.

The porous substrate has multiple pores to keep physiological saline. When measuring the Vmax, Tmax, and C5min, the porous substrate continuously receives physiological saline from the source of physiological saline, and thus, the pores of the surface remain saturated with physiological saline. If super absorbent polymer is spread on such a porous surface, the super absorbent polymer absorbs physiological saline filled in the pores, and simultaneously, the pores are filled with physiological saline fed from the source of physiological saline again. Namely, physiological saline continuously moves from the source of physiological saline to the porous substrate, and from the porous substrate to the super absorbent polymer, and at this time, by continuously measuring the amount of physiological saline that super absorbent polymer absorbs with the passage of time, the Vmax, Tmax, and C5min can be measured.

The porous substrate may be, for example, a glass filter, and specifically, a glass filter wherein a porosity according to ASTM standard is extra coarse.

The measurement of Vmax, Tmax, and C5min may be conducted, for example, by the property measuring apparatus of super absorbent polymer (100) as illustrated in Fig. 1. The property measuring apparatus of super absorbent polymer (100) may comprise the following constructions:
a scale(13);
a first water tank(10) placed on the scale(13);
a second water tank(20) placed around the scale, and covered with a glass filter(21) on the upper part;
a cylinder(22) placed on the glass filter(21) of the second water tank, equipped with mesh (not shown) on the lower surface contacting the glass filter(21), and having an open upper surface; and
a connection pipe(14) connecting the first water tank(10) with the second water tank(20), and transferring liquid from the first tank(10) to the second tank(20).

The first tank(10) feeds physiological saline(12) to the second water tank(20), and is placed on the scale(13). The scale(13) may be connected to a computer(PC) to continuously measure and record the weight of the first water tank(10).

The size of the first water tank(10) may be selected according to the amount of a sample (super absorbent polymer) used. For example, in case 0.1 g to 2.0 g of super absorbent polymer is used as a sample, as the first water tank(10), those having an internal volume of 500 cm³ or more, or 1000 cm³ or more, and 3000 cm³ or less, or 2000 cm³ or less may be used. Although the shape and size of the first water tank(10) are not specifically limited, one having a cylindrical shape with high height, or a rectangular shape may smoothly feed physiological saline(12) to the second water tank(20), and thus, is preferable. For example, as the first water tank(10), those having internal diameter and height of 8 cm or more, or 10 cm or more, and 30 cm or less, or 20 cm or less, respectively, may be used, but it is not limited thereto.

The first water tank(10) may be blocked on all sides, as shown in Fig. 1. Wherein an air passage pipe(11) may be installed to penetrate through the upper surface of the first water tank(10), so that the internal pressure of the first water tank(10) may be maintained at atmospheric pressure(760 ± 50 mmHg). Specifically, the pipe(11) may be installed such that the upper part may be exposed to the air, and the lower part may be submerged below the surface of physiological saline(12). When using the first water tank(10) of the above-explained size, the internal diameter of the pipe(11) may be about 3 to 6 mm, or 3 to 10 mm, but is not limited thereto. Further, the length of the pipe(11) may be appropriately controlled according to the depth of the water tank, and the like.

The first water tank(10) and the second water tank(20) are connected through a connection pipe(14), and thus, physiological saline(12) filled in the first water tank(10) may be continuously fed to the second water tank(20). In order to allow physiological saline to be safely fed to the second water tank(20) when measuring the properties of super absorbent polymer, the connection pipe(14) may be preferably located on the point less than half, or less than 1/3 of the height of each water tank.

The internal diameter of the connection pipe(14) may be, for example, in the range of 5 mm to 20 mm, or 10 to 15 mm. The diameter or length of the connection pipe may be appropriately controlled according to the location or size of the first water tank(10) and the second water tank(20).

As the second water tank(20), one having smaller internal volume than the first water tank(1 0) is used so that feeding of physiological saline from the first water tank may be sufficient during property measurement of super absorbent polymer. Preferably, the internal volume of the second water tank(20) may be 30% or more, or 50% or more, and 95% or less, or 90% or less, or 85% or less of the internal volume of the first water tank(1 0).

On the upper surface of the second water tank(20), a glass filter(21) is placed. On the glass filter, a cylinder(22) equipped with a mesh (not shown) on the lower surface, and having an open upper surface is placed, and a super absorbent polymer sample is spread on the mesh of the cylinder. The glass filter(21) receives physiological saline from the second water tank(20) to transfer it to the super absorbent polymer sample(23). If super absorbent polymer absorbs physiological saline of the glass filter, physiological saline moves from the first water tank to the second water tank, and to the glass filter again as much as absorbed, and the porous glass filter receives physiological saline from the second water tank that always maintains full water level, and remains saturated.

The glass filter(21) may constitute a part or the whole of the upper surface of the second water tank(20). The diameter of the glass filter may be appropriately selected according to the amount of a sample, and for example, in case 0.1 g to 2.0 g of super absorbent polymer is used as a sample, it is preferable that the diameter of the glass filter(21) may be 50 mm or more, or 70 mm or more, and 200 mm or less, or 150 mm or less, or 130 mm or less.

Further, it is preferable that the thickness of the glass filter(21) is 3 mm or more, or 5 mm or more and, 10 mm or less, or 9 mm or less, and when the porosity according to ASTM standard is extra coarse, physiological saline may be smoothly fed to super absorbent polymer during measurement, which is preferable.

The cylinder(22) is a part where a super absorbent polymer sample is spread, is equipped with a mesh on the lower surface, and has an open upper surface. The super absorbent polymer is spread inside the cylinder, namely, on the mesh of the lower surface, through the upper surface.

As the mesh, one having an opening of 100 µm or less, 80 µm or less, 60 µm or less, or 40 µm or less and, 25 µm or more may be used so that it may only function for supporting super absorbent polymer without absorbing physiological saline or affecting the movement of physiological saline. The thread diameter of the mesh, namely, the thickness of thread may be 25 µm or more, or 28 µm or more and, 80 µm or less, or 70 µm or less, or 50 µm or less.

As the material of the mesh, nylon or stainless, and the like may be used, but it is not limiter thereto, and various materials that have salt water resistance and does not have an influence on the property measurement of super absorbent polymer may be appropriately used.

Preferably, the internal diameter of the cylinder is not significantly different from the diameter of the glass filter, so that errors due to evaporation of physiological saline, and the like, may be reduced during property measurement. For example, a difference between the diameter of the cylinder and the diameter of the glass filter may be within 30%, or within 25%. The diameter of the cylinder may be identical to the diameter of the glass filter.

The length of the cylinder is not limited, but preferably, is 30 mm or more, or 40 mm or more and, 80 mm or less, or 60 mm or less, or 50 mm or less, so that super absorbent polymer may be uniformly spread.

The materials of the first water tank, second water tank, pipe, connection pipe, cylinder, and the like, constituting the apparatus, are not specifically limited, and those having salt water resistance may be appropriately used. Further, although the standard size of each part of the apparatus is illustrated above, it is presented only as illustration without limitations, and the standard size of each part may be appropriately controlled according to the amount of a sample used or the purpose of measurement.

Using the apparatus, absorption speed, absorption power, and diffusion performance of super absorbent polymer under no load may be conveniently and exactly measured.

The property measurement of super absorbent polymer using the above-explained property measuring apparatus may be specifically conducted through the following steps.
i) The cylinder is separated from the second water tank, and super absorbent polymer is spread inside the cylinder.
ii) Separately, the first water tank is filled with physiological saline(0.9 wt%, NaCl aqueous solution) so as to entirely fill the inside of the second water tank, thus saturating the glass filter of the second water tank with physiological saline.
iii) When the glass filter of the second water tank is saturated with physiological saline, the initial weight of the first water tank is measured, and then, the cylinder is put on the glass filter.
iv) The weight of the first water tank is continuously measured from the time the cylinder is put on the glass filter, thus obtaining data regarding the amount of physiological saline absorbed in the super absorbent polymer and the absorption speed.

The step i) is for the preparation of a sample. First, the cylinder is separated from the second water tank, and then, a super absorbent polymer sample is added through the upper surface of the cylinder, so that the super absorbent polymer is laid on the mesh of the lower surface. At this time, if super absorbent polymer particles overlap with each other, exact data cannot be obtained, and thus, they should be uniformly distributed.

The amount of the super absorbent polymer sample may be, for example, 0.1 g to 2.0 g, or 0.5 g to 2.0 g, or 1.0 g to 2.0 g. The particle diameter range of the sample is 150 µm to 850 µm.

The step ii) is for saturating the measurement apparatus with physiological saline, wherein physiological saline is fed to the first water tank so that the glass filter of the second water tank may be saturated. If the glass filter is saturated, iii) the initial weight of the first water tank is measured, and then, the cylinder where the super absorbent polymer is spread, is put on the glass filter so that the lower surface (namely, mesh side) may touch the glass filter.

In the measurement method, super absorbent polymer begins to absorb physiological saline from the moment the cylinder is put on the glass filter, physiological saline is fed from the first water tank to the second water tank as much as reduced accordingly, and the weight of the first water tank is reduced. Thus, the reduced weight of the first water tank may be considered as the amount(C) of physiological saline absorbed by super absorbent polymer.

Further, from the amount of physiological saline absorbed by super absorbent polymer per unit time, the absorption speed(V) of super absorbent polymer can be derived.

During the measurement, in each step, the outdoor air temperature of the apparatus and the temperature of physiological saline are maintained respectively in the range of 23°C to 25°C. In case the outdoor air temperature of the apparatus or the temperature of physiological saline does not fall within the above range, measurement errors may be generated.

Further, during the measurement, the outdoor air and the internal pressure of the first water tank are allowed to be maintained at atmospheric pressure (760 ± 50 mmHg). For example, by including a pipe as an air pathway on the upper part of the first water tank as explained above, the internal pressure may be maintained at atmospheric pressure.

After obtaining data regarding the amount of physiological saline absorbed by super absorbent polymer and the absorption speed under the above conditions, the maximum value of the absorption speed (Vmax), a time when the maximum value of the absorption speed (Tmax) is achieved, and the amount of physiological saline absorbed in the super absorbent polymer for 5 minutes (C5min) may be derived, and absorption force, absorption speed, and rewet and leakage prevention properties can be exactly measured or predicted therefrom.

### Preparation method of super absorbent polymer

Super absorbent polymer fulfilling the above properties may be prepared, for example, by a preparation method comprising steps of:
subjecting a monomer composition comprising water soluble ethylenically unsaturated monomers having acid groups, an internal crosslinking agent, and a polymerization initiator to polymerization, to form polymer in which the water soluble ethylenically unsaturated monomers and internal crosslinking agent are crosslinked (step 1);
grinding the polymer (step 2); and
drying and grinding the ground polymer to prepare super absorbent polymer particles (step 3),
wherein before grinding of the step 2, or drying of the step 3, a compound represented by the following Chemical Formula 1 or a salt thereof is added in the amount of 50 ppm to 7,000 ppm, based on the water soluble ethylenically unsaturated monomers.

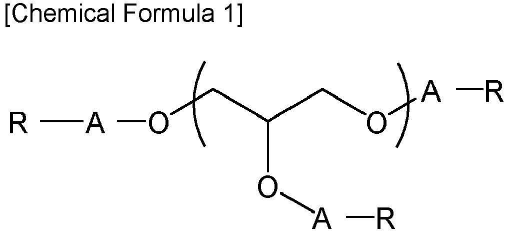

In the Chemical Formula 1,
A₁, A₂ and A₃ are each independently, a single bond, carbonyl, 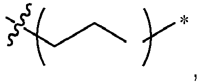 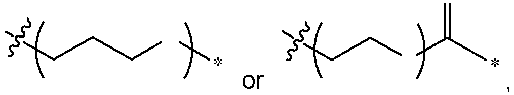 provided that one or more of them are carbonyl or 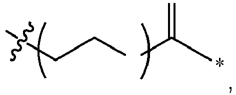 wherein m1, m2 and m3 are each independently, an integer of 1 to 8, each 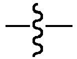 is connected to neighboring oxygen atom, and each 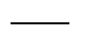 is connected to neighboring R₁, R₂ and R₃,
R₁, R₂ and R₃ are each independently, hydrogen, C6 to 18 linear or branched alkyl or C6 to 18 linear or branched alkenyl, and
n is an integer of 1 to 9.

The compound represented by the Chemical Formula 1 or a salt thereof may serve as a surfactant for polymer. If the surfactant is added before the polymer grinding or drying step, the surfactant may exist in large quantities on the surface of polymer having high moisture content, and high adhesion of polymer may be lowered, thus preventing excessive aggregation of polymer.

Further, the hydrophobic functional group parts included in the surfactant endow hydrophobicity to the surfaces of ground super absorbent polymer particles, to mitigate frictional force between the particles, thereby increasing apparent density of super absorbent polymer, and simultaneously, the hydrophilic functional group parts included in the surfactant are also bonded to the super absorbent polymer particles so that the surface tension of the polymer may not be lowered.

Further, by controlling the amount of the surfactant used to 50 ppm to 7,000 ppm based on the water soluble ethylenically unsaturated monomers, super absorbent polymer prepared may fulfill i) to iii) initial absorption speed and absorption power profiles as explained above.

Thereby, super absorbent polymer prepared by the above-explained preparation method may exhibit surface tension of equivalent level, compared to polymer prepared without using a surfactant, and simultaneously, have high apparent density value, and may exhibit excellent absorption speed and absorption capacity without rewet.

As used herein, the term "polymer" means a polymerized state of water soluble ethylenically unsaturated monomers, and it may include those of all moisture content ranges or particle diameter ranges.

Further, the term "super absorbent polymer" means crosslinked polymer, or base resin in the form of powder consisting of super absorbent polymer particles obtained by grinding of the crosslinked polymer, or it is used to include the crosslinked polymer or base resin made suitable for productization, for example, through drying, grinding, classification, surface crosslinking, and the like.

Further, the term "fine powder" means particles having particle diameter less than 150 µm. The particle diameter of such polymer particles may be measured according to EDANA (European Disposables and Nonwovens Association) Standard EDANA WSP 220.3 method.

Hereinafter, a method for preparing super absorbent polymer and according to one embodiment will be more specifically explained according to steps.

### Step of preparation and polymerization of monomer composition

The polymerization step may consist of mixing water soluble ethylenically unsaturated monomers having acid groups, an internal crosslinking agent and a polymerization initiator to prepare a monomer composition, and polymerizing the monomer composition to form polymer.

The water soluble ethylenically unsaturated monomers may be any monomers commonly used in the preparation of superabsorbent polymer. As non-limiting examples, the water soluble ethylenically unsaturated monomer may be a compound represented by the following Chemical Formula 2:

[Chemical Formula 2] R-COOM'

In the Chemical Formula 2,
R is a C2-5 alkyl group comprising an unsaturated bond,
M' is a hydrogen atom, a monovalent or divalent metal, an ammonium group or an organic amine salt.

Preferably, the monomers may be one or more selected from the group consisting of (meth)acrylic acid, and monovalent (alkali) metal salts, divalent metal salts, ammonium salts and organic amine salts thereof.

In case (meth)acrylic acid or a salt thereof is used as the water soluble ethylenically unsaturated monomer, superabsorbent polymer with improved absorption property can be obtained, which is preferable. In addition, as the monomers, one or more selected from the group consisting of maleic anhydride, fumaric acid, crotonic acid, itaconic acid, 2-acryloylethane sulfonic acid, 2-methacryloylethane sulfonic acid, 2-(meth)acryloylpropane sulfonic acid, or 2-(meth)acrylamide-2-methylpropane sulfonic acid, (meth)acrylamide, N-substituted (meth)acrylate, 2-hydroxyethyl (meth)acrylate, 2-hydroxypropyl (meth)acrylate, methoxy polyethylene glycol (meth)acrylate or polyethylene glycol (meth)acrylate, (N,N)-dimethylaminoethyl (meth)acrylate or (N,N)-dimethylaminopropyl (meth)acrylamide may be used.

The water soluble ethylenically unsaturated monomers having acid groups may be partially neutralized with alkali substance such as sodium hydroxide, potassium hydroxide, ammonium hydroxide, and the like, before being used for polymerization. Specifically, in the step of preparing a monomer composition, the alkali substance may be added as a neutralization agent so that at least a part of the acid groups of the water soluble ethylenically unsaturated monomers may be neutralized, before initiating polymerization.

In case monomers are neutralized before used, the degree of neutralization of the monomer may be 40 to 95 mol%, or 40 to 80 mol%, or 45 to 75 mol%. Although the range of the neutralization degree may vary according to the final properties, if the neutralization degree is too high, neutralized monomers may be precipitated, and thus, it may be difficult to smoothly progress polymerization, and to the contrary, if the neutralization degree is too low, absorption force of polymer may be significantly lowered, and it may exhibit elastic rubber-like properties, which is difficult to handle.

Alternatively, the water soluble ethylenically unsaturated monomers may be used for polymerization while the acid groups are not neutralized.

The water soluble ethylenically unsaturated monomers (for example, acrylic acid), of which acid groups are not neutralized, are liquid and have high miscibility with a solvent (water), and thus, exist in the state of a mixed solution in the monomer composition. However, water soluble ethylenically unsaturated monomers, of which acid groups are neutralized, are solid at room temperature, and have different solubilities according to the temperature of a solvent (water), and the solubility is lower as the temperature is lower.

As such, the water soluble ethylenically unsaturated monomers (for example, acrylic acid), of which acid groups are not neutralized, have higher solubility to or miscibility with a solvent (water) than the monomers of which acid groups are neutralized, and are not extracted even at low temperature, and thus, are favorable for polymerization for a long time at low temperature. Thus, by conducting polymerization for a long time using the water soluble ethylenically unsaturated monomers (for example, acrylic acid), of which acid groups are not neutralized, polymer having higher molecular weight and uniform molecular weight distribution may be stably formed.

Further, in case water soluble ethylenically unsaturated monomers of which acid groups are not neutralized are used, polymer of longer chain can be formed, thus achieving the effect for reducing extractable contents that exist in non-crosslinked state due to incomplete polymerization or crosslinking.

As such, in case polymerization is first conducted while the acid groups of monomers are not neutralized, a neutralizing agent may be added to polymer after the polymerization step, to neutralize at least a part of the acid groups.

Wherein, as the neutralizing agent, the above-explained alkali substance such as sodium hydroxide, potassium hydroxide, ammonium hydroxide, and the like may be used.

Further, a degree of neutralization, which refers to a degree of neutralization of the acid groups included in the polymer by the neutralizing agent, may be 50 to 90 mol%, or, 60 to 85 mol%, or 65 to 85 mol%, or 65 to 75 mol%. Although the range of the neutralization degree may vary according to the final properties, if the neutralization degree is too high, absorption power of super absorbent polymer may decrease, and the concentration of carboxyl groups on the particle surface may be too low, and thus, it may be difficult to sufficiently conduct surface crosslinking in the subsequent process, and thus, absorption property under load or permeability may decrease. To the contrary, if the neutralization degree is too low, absorption force of polymer may be significantly lowered, and it may exhibit elastic rubber-like properties, which is difficult to handle.

Meanwhile, in the monomer composition, the concentration of the water soluble ethylenically unsaturated monomers may be appropriately controlled considering polymerization time and reaction conditions, and the like, and it may be controlled to about 20 to about 60 wt%, or about 20 to about 40 wt%, regardless of whether neutralized or not.

As used herein, the term 'internal crosslinking agent' is used to distinguish from a surface crosslinking agent for crosslinking the surface of super absorbent polymer particles described later, and it functions for introducing crosslinks between unsaturated bonds of the above-explained water soluble ethylenically unsaturated monomers, to form polymer comprising a crosslink structure.

In this step, crosslinking is progressed without distinction of a surface and inside, but in case a surface crosslinking process of super absorbent polymer is progressed as described later, the surface of the finally prepared super absorbent polymer particles may include a structure newly crosslinked by the surface crosslinking agent, and the inside of the super absorbent polymer particles may maintain a structure crosslinked by the internal crosslinking agent.

According to one embodiment of the present disclosure, as the internal crosslinking agent, one or more of multifunctional acrylate-based compounds, multifunctional allyl-based compounds, or multifunctional vinyl-based compounds may be used.

As non-limiting examples of the multifunctional acrylate-based compounds, ethyleneglycol di(meth)acrylate, diethyleneglycol di(meth)acrylate, triethyleneglycol di(meth)acrylate, tetraethyleneglycol di(meth)acrylate, polyethyleneglycol di(meth)acrylate, propyleneglycol di(meth)acrylate, tripropyleneglycol di(meth)acrylate, polypropyleneglycol di(meth)acrylate, butanediol di(meth)acrylate, butyleneglycol di(meth)acrylate, hexanediol di(meth)acrylate, pentaerythritol di(meth)acrylate, pentaerythritol tri(meth)acrylate, pentaerythritol tetra(meth)acrylate, dipentaerythritol di(meth)acrylate, dipentaerythritol tri(meth)acrylate, dipentaerythritol tetra(meth)acrylate, dipentaerythritol penta(meth)acrylate, trimethylolpropane di(meth)acrylate, trimethylolpropane tri(meth)acrylate, glycerin di(meth)acrylate, and glycerin tri(meth)acrylate, and the like may be mentioned, and one of them or two or more kinds of them may be mixed and used.

As non-limiting examples of the multifunctional allyl-based compounds, ethyleneglycol diallyl ether, diethyleneglycol diallyl ether, triethyleneglycol diallyl ether, tetraethyleneglycol diallyl ether, polyethyleneglycol diallyl ether, propyleneglycol diallyl ether, tripropyleneglycol diallyl ether, polypropyleneglycol diallyl ether, butanediol diallyl ether, butyleneglycol diallyl ether, hexanediol diallyl ether, pentaerythritol diallyl ether, pentaerythritol triallyl ether, pentaerythritol tetraallyl ether, dipentaerythritol diallyl ether, dipentaerythritol triallyl ether, dipentaerythritol tetraallyl ether, dipentaerythritol pentaallyl ether, trimethylolpropane diallyl ether, trimethylolpropane triallyl ether, glycerin diallyl ether, and glycerin triallyl ether, and the like may be mentioned, and one of them or two or more kinds of them may be mixed and used.

As non-limiting examples of the multifunctional vinyl-based compounds, ethyleneglycol divinyl ether, diethyleneglycol divinyl ether, triethyleneglycol divinyl ether, tetraethyleneglycol divinyl ether, polyethyleneglycol divinyl ether, propyleneglycol divinyl ether, tripropyleneglycol divinyl ether, polypropyleneglycol divinyl ether, butanediol divinyl ether, butyleneglycol divinyl ether, hexanediol divinyl ether, pentaerythritol divinyl ether, pentaerythritol trivinyl ether, pentaerythritol tetravinyl ether, dipentaerythritol divinyl ether, dipentaerythritol trivinyl ether, dipentaerythritol tetravinyl ether, dipentaerythritol pentainyl ether, trimethylolpropane divinyl ether, trimethylolpropane trivinyl ether, glycerin divinyl ether, and glycerin trivinyl ether, and the like may be mentioned, and one of them or two or more kinds of them may be mixed and used. Preferably, pentaerythritol triallyl ether may be used.

In the above explained multifunctional allyl-based compounds, or multifunctional vinyl-based compounds, two or more unsaturated groups included in the molecule respectively bind to the unsaturated bonds of water soluble ethylenically unsaturated monomers, or unsaturated bonds of other internal crosslinking agents, thus forming a crosslink structure during the polymerization process, and unlike acrylate-based compounds comprising an ester bond(-(C=O)O-) in the molecule, the crosslink may be more stably maintained even during the neutralization process after the polymerization reaction.

Thus, gel strength of prepared super absorbent polymer may increase, process stability during a discharge process after polymerization may increase, and extractable contents may be minimized.

The crosslinking polymerization of water soluble ethylenically unsaturated monomers in the presence of such an internal crosslinking agent may be conducted in the presence of a thickener, a plasticizer, a preservative, an antioxidant, and the like, as necessary.

In the monomer composition, the internal crosslinking agent may be used in an amount of 0.01 to 5 parts by weight, based on 100 parts by weight of the water soluble ethylenically unsaturated monomers. For example, the internal crosslinking agent may be used in an amount of 0.01 parts by weight or more, or 0.05 parts by weight or more, or 0.1 parts by weight or more, and 5 parts by weight or less, or 3 parts by weight or less, or 2 parts by weight or less, or 1 part by weight or less, or 0.7 parts by weight or less, based on 100 parts by weight of the water soluble ethylenically unsaturated monomers. If the content of the internal crosslinking agent is too low, crosslinking may not sufficiently occur, and thus, it may be difficult to realize strength above an optimum level, and if the content of the internal crosslinking agent is too high, the internal crosslinking density may increase, and thus, it may be difficult to realize desired water retention capacity.

The polymer formed using such an internal crosslinking agent has a three-dimensional network structure in which main chains formed by polymerization of the water soluble ethylenically unsaturated monomers are crosslinked by the internal crosslinking agent. As such, in case polymer has a three-dimensional network structure, compared to a two-dimensional linear structure that is not additionally crosslinked by an internal crosslinking agent, the properties of super absorbent polymer such as water retention capacity and absorbency under pressure may be remarkably improved.

According to one embodiment of the present disclosure, the step of conducting polymerization of the monomer composition to form polymer may be conducted in a batch type reactor.

In the common preparation method of super absorbent polymer, the polymerization method is largely divided into thermal polymerization and photopolymerization according to a polymerization energy source, and commonly, thermal polymerization may be progressed in a reactor equipped with a stirring axis such as a kneader, and photopolymerization may be progressed in a reactor equipped with a movable conveyor belt or in a flat-bottom container.

Meanwhile, by such a polymerization method, generally, polymer having modest molecular weight and wide molecular weight distribution is formed according to a short polymerization reaction time (for example, 1 hour or less).

Meanwhile, in case photopolymerization is progressed in a reactor equipped with a movable conveyor belt or in a flat bottom container, a hydrogel polymer sheet having a width of the belt is commonly obtained, and the thickness of the polymer sheet may vary according to the concentration of introduced monomer composition and introduction speed or introduction amount, but commonly, it may be about 0.5 to about 5cm.

However, in case the monomer composition is supplied such that the thickness of the polymer sheet becomes too thin, production efficiency may be low, and in case the thickness of polymer sheet is increased for productivity, a polymerization reaction may not uniformly occur over the entire thickness, and thus, it may be difficult to form high quality polymer.

Further, since polymerization in the reactor equipped with a stirring axis and conveyor belt is continuously progressed while polymerization product moves and new monomer composition is fed to the reactor, polymers having different polymerization rates may be mixed, and thus, polymerization may not uniformly occur over the entire monomer composition, thus causing property deterioration.

However, according to one embodiment of the present disclosure, by progressing fixed-bed type polymerization in a batch type reactor, there is little concern about mixing of polymers having different polymerization rates, and thus, polymer having uniform quality may be obtained.

Further, the polymerization step is conducted in a batch type reactor having a predetermined volume, and a polymerization reaction is conducted for a longer time than the case of conducting continuous polymerization in a reactor equipped with a conveyor belt, for example, for 3 hours or more. Despite the long polymerization reaction time, since polymerization is conducted for non-neutralized water soluble ethylenically unsaturated monomers, even if polymerization is conducted for a long time, monomers may not be easily precipitated, thus favorable for polymerization for a long time.

Meanwhile, the polymerization in a batch type reactor of the present disclosure uses thermal polymerization, and thus, uses a thermal polymerization initiator as the polymerization initiator.

As the thermal polymerization initiator, one or more selected from the group consisting of persulfate-based initiators, azo-based initiators, hydrogen peroxide and ascorbic acid may be used. Specifically, as the examples of the persulfate-based initiators, sodium persulfate (Na₂S₂O₈), potassium persulfate (K₂S₂O₈), ammonium persulfate ((NH₄)₂S₂O₈), and the like, may be mentioned, and as the examples of the azo-based initiators, 2,2-azobis(2-amidinopropane) dihydrochloride, 2,2-azobis-(N,N-dimethylene)isobutyramidine dihydrochloride, 2-(carbamoylazo)isobutylonitrile, 2,2-azobis[2-(2-imidazolin-2-yl)propane] dihydrochloride, 4,4-azobis-(4-cyanovaleric acid), and the like may be mentioned. More various thermal polymerization initiators are stated in Odian, 'Principle of polymerization (Wiley, 1981)', p203, but the present disclosure is not limited thereto.

Such a polymerization initiator may be added in the amount of 2 parts by weight or less, based on 100 parts by weight of the water soluble ethylenically unsaturated monomers. If the concentration of the polymerization initiator is too low, polymerization speed may become slow, and remaining monomers may be extracted in a large quantity in the final product. To the contrary, if the concentration of the polymerization initiator is higher than the above range, polymer chains making up the network of superabsorbent polymer may become short, and thus, extractable contents may increase, and absorbency under pressure of superabsorbent polymer may be lowered, thereby deteriorating the properties of polymer.

Meanwhile, according to one embodiment, polymerization may be initiated by introducing the initiator together with a reducing agent forming a redox couple with the initiator.

Specifically, the initiator and reducing agent, when introduced in a polymerization solution, react with each other to form radicals.

The radicals formed react with monomers, and since the oxidation-reduction reaction between the initiator and reducing agent is highly reactive, even if small amounts of initiator and reducing agent are used, polymerization may be initiated, thus enabling low temperature polymerization without need to increase process temperature, and minimizing property change of the polymer solution.

The polymerization reaction using an oxidation-reduction reaction may smoothly occur at a temperature around a room temperature (25 °C) or lower temperature. For example, the polymerization reaction may be conducted at a temperature of 5 °C or more and 25 °C or less, or 5 °C or more and 20 °C or less.

In one embodiment of the present disclosure, in case a persulfate-based initiator is used as the initiator, as the reducing agent, one or more selected from the group consisting of sodium metabisulfite (Na₂S₂O₅); tetramethyl ethylenediamine (TMEDA); a mixture of iron sulfate(II) and EDTAFeSO₄/EDTA); sodium formaldehyde sulfoxylate; and disodium 2-hydroxy-2-sulfinoacteate may be used.

For example, potassium persulfate may be used as the initiator, and disodium 2-hydroxy-2-sulfinoacetate may be used as the reducing agent; ammonium persulfate may be used as the initiator, and tetramethyl ethylenediamine may be used as the reducing agent; or sodium persulfate may be used as the initiator, and sodium formaldehyde sulfoxylate may be used as the reducing agent.

In another embodiment of the present disclosure, in case a hydrogen peroxide-based initiator is used as the initiator, the reducing agent may be one or more selected from the group consisting of ascorbic acid; sucrose; sodium sulfite (Na₂SO₃) sodium metabisulfite (Na₂S₂O₅); tetramethyl ethylenediamine (TMEDA); a mixture of iron sulfate(II) and EDTA (FeSO₄/EDTA); sodium formaldehyde sulfoxylate; disodium 2-hydroxy-2-sulfinoacteate; and disodium 2-hydroxy-2-sulfoacteate.

The monomer composition may further comprise additives such as a thickener, a plasticizer, a preservative, an antioxidant, and the like, as necessary.

Further, the monomer composition may be prepared in the form of a solution dissolved in a solvent such as water, and the solid content in the solution of the monomer composition, i.e., the concentration of monomers, internal crosslinking agent and polymerization initiator may be appropriately controlled considering polymerization and reaction conditions, and the like. For example, solid content in the monomer composition may be 10 to 80 wt%, or 15 to 60 wt%, or 30 to 50 wt%.

Wherein, a solvent that can be used is not limited as long as it can dissolve the above explained components, and for example, water, ethanol, ethyleneglycol, diethyleneglycol, triethyleneglycol, 1,4-butanediol, propyleneglycol, ethyleneglycol monobutyl ether, propyleneglycol monomethyl ether, propyleneglycol monomethyl ether acetate, methylethylketone, acetone, methylamylketone, cyclohexanone, cyclopentanone, diethyleneglycol monomethyl ether, diethyleneglycol ethyl ether, toluene, xylene, butyrolactone, carbitol, methyl celosolve acetate and N,N-dimethylacetamide or mixtures thereof may be used.

Polymer obtained by such a method may have high molecular weight and uniform molecular weight distribution, and reduced extractable contents, because polymerization is conducted using non-neutralized ethylenically unsaturated monomers.

Polymer obtained by such a method is in the state of hydrogel polymer and may have a moisture content of 30 to 80 wt%. For example, the moisture content of the polymer may be 30 wt% or more, or 45 wt% or more, or 50 wt% or more, and 80 wt% or less, or 70 wt% or less, or 60 wt% or less.

If the moisture content of polymer is too low, it may be difficult to secure appropriate surface area in the subsequent grinding step, and thus, the polymer may not be effectively ground, and if the moisture content of polymer is too high, pressure applied in the subsequent grinding step may increase, and thus, it may be difficult to grind to a desired particle size.

Meanwhile, throughout the specification, a "moisture content" is the content of moisture occupied, based on the total polymer weight, and it means a value obtained by subtracting the weight of dried polymer from the weight of polymer. Specifically, it is calculated by measuring weight decrease according to evaporation of moisture in the polymer while increasing the temperature of polymer in the state of crumb to dry through infrared heating. Wherein, a temperature is increased from a room temperature to about 180°C, and then, maintained at 180°C, and the total drying time is set to 40 minutes including 5 minutes of the temperature rise step.

### Step of griding polymer

Next, a step of grinding hydrogel polymer having a moisture content of 30 to 80 wt% is conducted. During this process, fine cutting and aggregation of polymer may be simultaneously conducted, and secondary aggregated particles obtained by aggregation of finely cut polymer may have significantly increased surface area, and thus, have remarkably improved absorption speed.

The step of grinding polymer may be conducted after adding a compound represented by the above Chemical Formula 1 or a salt thereof to polymer.

The compound represented by the Chemical Formula 1 or a salt thereof is used as a surfactant for inhibiting aggregation of super absorbent polymer particles.

The surfactant represented by the Chemical Formula 1 is nonionic surfactant, and has excellent surface adsorption performance by hydrogen bonding force even with non-neutralized polymer, and thus, is suitable for realizing the aimed aggregation controlling effect. To the contrary, in case anionic surfactant is mixed with polymer neutralized with a neutralization agent such as NaOH, Na₂SO₄, and the like instead of nonionic surfactant, it is adsorbed by Na+ ions to the carboxyl substituent group of polymer, and in case it is mixed with non-neutralized polymer, due to competition with anions of the carboxyl substituent group of polymer, adsorption efficiency to polymer may be relatively lowered.

Specifically, in the surfactant represented by the Chemical Formula 1, hydrophobic functional groups are end functional groups R₁, R₂, R₃ parts (in case they are not hydrogen), and hydrophilic functional groups further include a part derived from glycerol in the chain, and end hydroxyl group (in case Aₙ is a single bond, and simultaneously, Rₙ is hydrogen, n=1~3), wherein the glycerol-derived part and end hydroxyl group are hydrophilic functional groups and perform a function for improving adsorption performance to the polymer surface. Thereby, aggregation of super absorbent polymer particles may be effectively inhibited.

In the Chemical Formula 1, hydrophobic functional groups R₁, R₂, Rs(in case they are not hydrogen) are each independently, C6 to 18 linear or branched alkyl or C6 to 18 linear or branched alkenyl. Wherein, in case R₁, R₂, R₃ (in case they are not hydrogen) are alkyl or alkenyl having a carbon number less than 6, due to short chain length, aggregation controlling effect may not be effectively achieved, and in case R₁, R₂, R₃ (in case they are not hydrogen) are alkyl or alkenyl having a carbon number greater than 18, mobility of the surfactant may decrease, and thus, it may not be effectively mixed with polymer, and due to cost rise of surfactant, unit cost of a composition may increase.

Preferably, in case each of R₁, R₂, R₃ is hydrogen or C6 to 18 linear or branched alkyl, it may be 2-methylhexyl, n-heptyl, 2-methylheptyl, n-octyl, n-nonyl, n-decanyl, n-undecanyl, n-dodecanyl, n-tridecanyl, n-tetradecanyl, n-pentadecanyl, n-hexadecanyl, n-heptadecanyl, or n-octadecanyl, or in case it is C6 to 18 linear or branched alkenyl, it may be 2-hexenyl, 2-heptenyl, 2-octenyl, 2-nonenyl, n-decenyl, 2-undecenyl, 2-dodecenyl, 2-tridecenyl, 2-tetradecenyl, 2-pentadecenyl, 2-hexadecenyl, 2-heptadecenyl, or 2-octadecenyl.

The surfactant may be selected from compounds represented by the following Chemical Formula 1-1 to Chemical Formula 1-14:

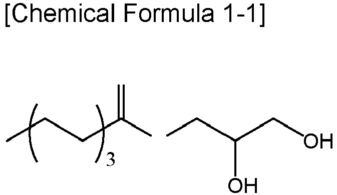

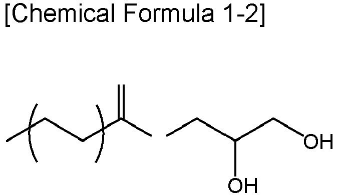

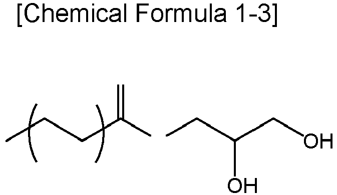

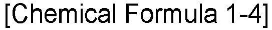

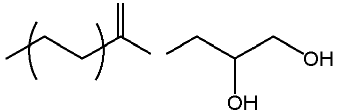

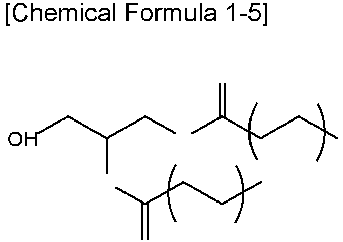

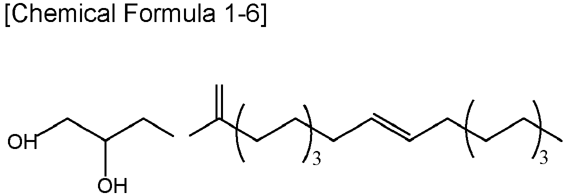

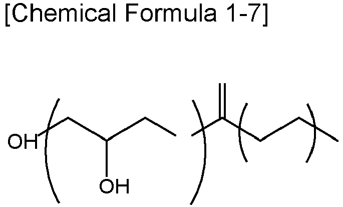

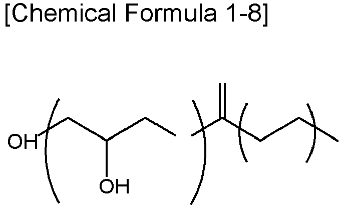

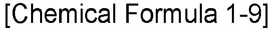

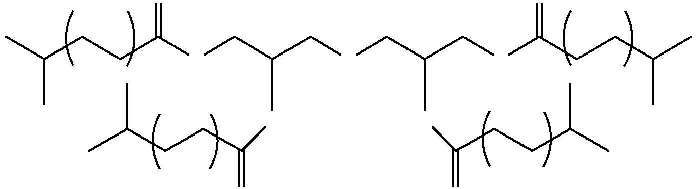

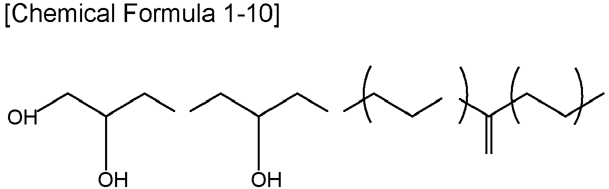

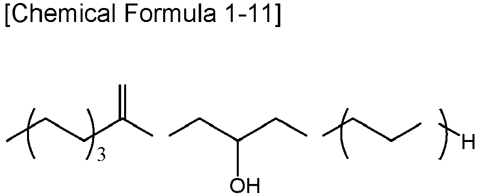

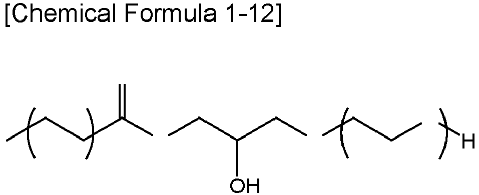

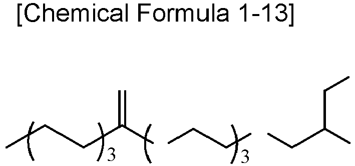

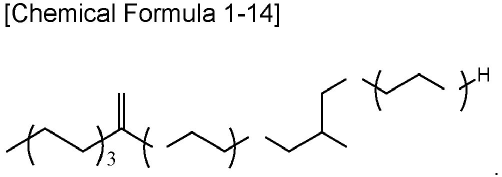

Meanwhile, the surfactant may be used in the amount of 50 ppm to 7,000 ppm, based on the water soluble ethylenically unsaturated monomers. If the amount of the surfactant used is too small, it may not be uniformly adsorbed on polymer surface, and thus, reaggregation of particles may be generated after grinding or drying, and if the amount of the surfactant used is too large, the properties of the finally prepared super absorbent polymer may be deteriorated. For example, the surfactant may be used in an amount of 50 ppm or more, 100 ppm or more, 150 ppm or more, or 1,000 ppm or more and, 7,000 ppm or less, 6,000 ppm or less, 5,000 ppm or less, 4,000 ppm or less, or 3,000 ppm or less, based on the water soluble ethylenically unsaturated monomers.

A method for mixing such a surfactant with polymer is not specifically limited as long as it can uniformly mix them. Specifically, the surfactant may be dry mixed, or it may be dissolved in a solvent and then mixed in a solution state, or it may be molten and then mixed.

For example, the surfactant may be mixed in the state of a solution dissolved in a solvent. Wherein, as the solvent, inorganic solvents or organic solvents may be used without limitations, but considering easiness of a drying process and cost of solvent recovery system, water is most appropriate. Further, the solution and polymer may be put in a reactor and mixed, or polymer may be put in a mixer and the solution may be sprayed, or polymer and the solution may be continuously supplied to a continuously operated mixer and mixed, and the like.

In case monomers in which a part of the acid groups are neutralized are used in the step 1, step 2 is followed by step 1.

Meanwhile, in case non-neutralized monomers are used in the step 1, a step of neutralizing at least a part of the acid groups included in the polymer may be conducted as explained above, and the neutralization step and the polymer grinding step may be simultaneously or sequentially progressed.

Specifically, a neutralization agent may be added to polymer to neutralize the acid groups first, and then, a surfactant may be added to the neutralized polymer and the polymer mixed with the surfactant may be ground, or a neutralization agent and a surfactant may be simultaneously added to polymer to conduct neutralization and grinding of polymer. Alternatively, a surfactant may be added first and a neutralization agent may be added later. Alternatively, a neutralization agent and a surfactant may be alternately added. Alternatively, a surfactant may be added first and ground, and then, a neutralization agent may be added to neutralize, and a surfactant may be additionally added to the neutralized hydrogel polymer and a grinding process may be additionally conducted. Meanwhile, for uniform neutralization over the entire polymer, it may be preferable to set a regular time difference between the introduction of the neutralization agent and the grinding process.

At least a part or significant amount of the surfactant may exist on the surfaces of the hydrogel polymer particles.

Wherein, the description "the surfactant exists on the surfaces of hydrogel polymer particles" means that at least a part or significant amount of the surfactant is adsorbed or bonded to the surfaces of the hydrogel super absorbent polymer particles. Specifically, the surfactant may be physically or chemically adsorbed to the surface of the super absorbent polymer. More specifically, the hydrophilic functional groups of the surfactant may be physically adsorbed to the hydrophilic parts of the super absorbent polymer surface by intermolecular force such as dipole-dipole interaction. As such, the hydrophilic parts of the surfactant are physically adsorbed to the surface of the super absorbent polymer particles to cover the surface, and the hydrophobic parts of the surfactant are not adsorbed to the surface of polymer particles, and thus, polymer particles may be coated with the surfactant in the form of a micelle structure. This is because the surfactant is not added during the polymerization process of the water soluble ethylenically unsaturated monomers, but added in the grinding step after formation of polymer, and compared to the case wherein the surfactant is added during the polymerization process and exists inside the polymer, a function as a surfactant may be fully performed, and since grinding and aggregation simultaneously occur, particles having large surface area in the form of aggregated fine particles may be obtained.

The grinding step may be conducted two or more times.

The kind of grinder used for the grinding is not specifically limited, and specifically, it may include one selected from the group consisting of a vertical pulverizer, a turbo cutter, a turbo grinder, a rotary cutter mill, a cutter mill, a disc mill, a shred crusher, a crusher, a chopper and a disc cutter.

The grinding according to one embodiment may be conducted by a grinding apparatus comprising: a body part comprising a transfer space to which polymer is transferred inside; a screw member that is rotationally installed in the transfer space and moves polymer; a driving motor that provides rotational driving force to the screw member; a cutter member that is installed in the body part and grinds the polymer; and a porous plate that discharges the polymer ground by the cutter member to the outside of the body part, and is equipped with multiple holes. Wherein, the size of holes equipped in the porous plate may be 1 mm to 20 mm, or 5 mm to 15 mm, or 5 mm to 12 mm.

In case grinding is progressed while controlling the aggregation of the polymer using such a grinding device, smaller particle size distribution (particle diameter of tens to hundreds of micrometers) may be realized, and thus, the subsequent drying and grinding process may be conducted under milder conditions, thereby preventing generation of fine powders and improving the properties of super absorbent polymer.

### Step of drying and grinding polymer

Next, the ground polymer is dried and ground to prepare super absorbent polymer particles.

Wherein, before conducting the drying step, a compound represented by the Chemical Formula 1 or a salt thereof may be added as a surfactant.

To the surfactant, the above explanations are applied.

The drying step may be conducted such that the moisture content of super absorbent polymer may become 20 wt% or less, or 15 wt% or less and, 1 wt% or more, or 3 wt% or more.

For this purpose, a temperature in a dryer used in the drying step may be about 150°C or less, for example, about 80°C to about 150°C, and thus, the drying may be conducted at relatively low temperature. If the temperature in a dryer is too low, a drying time may be too lengthened, and if the drying temperature is too high, super absorbent polymer having lower moisture content than desired may be obtained.

Wherein, drying may be conducted by moving type drying wherein materials move while dried, or fixed-bed type drying wherein materials do not move.

The moving type drying refers to a method of drying while mechanically stirring a dried body. Wherein, a direction where hot air passes through material may be identical to or different from the circulation direction of the material. Alternatively, material may be circulated inside a dryer, and heat transfer fluid may be passed through a separate pipe outside the dryer to dry the material.

The fixed-bed type drying refers to a method of drying wherein hot air passes through material from the top to the bottom, while material to be dried is stopped on a bottom such as a perforated steel plate through which air can pass.

Next, the dried super absorbent polymer particles are ground to prepare super absorbent polymer particles.

Specifically, the grinding step may be conducted by grinding dried super absorbent polymer particles to a particle size of normal particle level, namely, particle diameter of 150µm to 850µm.

A grinder used for this purpose may be specifically, a vertical pulverizer, a turbo cutter, a turbo grinder, a rotary cutter mill, a cutter mill, a disc mill, a shred crusher, a crusher, a chopper or a disc cutter, and the like, but is not limited thereto.

Further, as a grinder, a pin mill, a hammer mill, a screw mill, a roll mill, a disc mill or a jog mill, and the like may be also used, but is not limited thereto.

### Additional steps

After the step of drying and grinding super absorbent polymer particles, a step of classifying the ground super absorbent polymer particles according to particle diameter may be further included.

Further, after grinding and/or classifying super absorbent polymer particles, a step of forming surface crosslink layers on at least a part of the surfaces of the super absorbent polymer particles in the presence of a surface crosslinking agent, may be further included. By this step, crosslinked polymer included in the super absorbent polymer particle is additionally crosslinked by the surface crosslinking agent, thus forming surface crosslink layers on at least a part of the surfaces of the super absorbent polymer particles.

As the surface crosslinking agent, those previously used for the preparation of super absorbent polymer may be used without specific limitations. For example, the surface crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol and glycerol; one or more carbonate-based compounds selected from the group consisting of ethylene carbonate, propylene carbonate and glycerol carbonate; epoxy compounds such as ethyleneglycol diglycidyl ether, and the like; oxazoline compounds such as oxazolidinone, and the like; polyamine compounds; oxazoline compounds; mono-, di- or polyoxazolidinone compounds; or cyclic urea compounds, and the like.

Specifically, as the surface crosslinking agent, one or more kinds, or two or more kinds, or three or more kinds of the above-explained surface crosslinking agents may be used, and for example, ethylene carbonate-propylene carbonate (ECPC), propylene glycol and/or glycerol carbonate may be used.

Such a surface crosslinking agent may be used in the amount of about 0.001 to about 5 parts by weight, based on 100 parts by weight of the super absorbent polymer particles. For example, the surface crosslinking agent may be used in the content of 0.005 parts by weight or more, or 0.01 parts by weight or more, or 0.05 parts by weight or more, and or 5 parts by weight or less, or 4 parts by weight or less, or 3 parts by weight or less, based on 100 parts by weight of the super absorbent polymer particles. By controlling the content range of the surface crosslinking agent within the above-explained range, super absorbent polymer exhibiting excellent absorption properties may be prepared.

Further, the step of forming a surface crosslink layer may be conducted with adding inorganic substance to the surface crosslinking agent. Namely, in the presence of the surface crosslinking agent and inorganic substance, the surfaces of the super absorbent polymer particles may be additionally crosslinked to form surface crosslink layers.

As such inorganic substance, one or more selected from the group consisting of silica, clay, alumina, silica-alumina composite material, titania, zinc oxide and aluminum sulfate may be used. The inorganic substance may be used in the form of powder or liquid, and particularly, alumina powder, silica-alumina powder, titania powder, or nano silica solution may be used. Further, the inorganic substance may be used in the content of about 0.001 to about 1 part by weight, based on 100 parts by weight of the super absorbent polymer particles.

Further, a method for mixing the surface crosslinking agent with a super absorbent polymer composition is not limited. For example, the surface crosslinking agent and super absorbent polymer composition may be put in a reactor and mixed, or the surface crosslinking agent may be sprayed to the super absorbent polymer composition, or the super absorbent polymer composition and surface crosslinking agent may be continuously fed to a continuously operated mixer and mixed.

When mixing the surface crosslinking agent with the super absorbent polymer composition, water and methanol may be mixed and additionally added. In case water and methanol are added, the surface crosslinking agent may be uniformly dispersed in the super absorbent polymer composition. Wherein, the content of water and methanol added may be appropriately controlled to induce uniform dispersion of the surface crosslinking agent, prevent agglomeration of the super absorbent polymer composition, and simultaneously, optimize the surface penetration depth of the crosslinking agent.

The surface crosslinking process may be conducted at a temperature of about 80°C to about 250°C. More specifically, the surface crosslinking process may be conducted at a temperature of about 100°C to about 220°C, or about 120°C to about 200°C, for about 20 minutes to about 2 hours, or about 40 minutes to about 80 minutes. When fulfilling the above-explained surface crosslinking process conditions, the surfaces of super absorbent polymer particles may be sufficiently crosslinked, and thus, absorbency under pressure may increase.

Temperature rising means for the surface crosslinking reaction are not specifically limited. A heating medium may be supplied, or a heat source may be directly supplied to heat. Wherein, as the heating medium that can be used, temperature-raised fluids such as steam, hot air, hot oil, and the like may be used, but it is not limited thereto, and the temperature of the heating medium may be appropriately selected considering the means of heating medium, temperature rise speed, and target temperature to be increased. Meanwhile, as the directly supplied heat source, electric heating, gas heating may be mentioned, but it is not limited thereto.

According to one embodiment of the present disclosure, after the step of forming surface crosslink layers on at least a part of the surfaces of super absorbent polymer particles, one or more of a cooling step of the super absorbent polymer particles having surface crosslink layers, a hydration step of adding water to the super absorbent polymer particles having surface crosslink layers, and a post-treatment step of adding additives to the super absorbent polymer particles having surface crosslink layers, may be conducted. Wherein, the cooling step, hydration step, or post-treatment step may be sequentially or simultaneously conducted.

The additives added in the post-treatment step may include a permeability improver, an anti-caking agent, a flow improver, and an anti-oxidant, and the like, but are not limited thereto.

By selectively conducting the cooling step, hydration step, and post-treatment step, the moisture content of the final super absorbent polymer can be improved, and a super absorbent polymer product with higher quality can be prepared.

Hereinafter, preferable examples will be presented to assist in understanding of the present disclosure, but the following examples are presented only as the illustrations of the present disclosure, and it is obvious to one of ordinary knowledge in the art that various alterations and modifications can be made within the scope of the categories and technical ideas of the present disclosure, and such alterations and modifications belong to the claims attached.

### [Example]

### Comparative Example 1

### (Polymer preparation step)

In a glass reactor, 488.5 g of acrylic acid, 663.5 g of 31.5 wt% NaOH aqueous solution, and 375 g of water were mixed to prepare a neutralized solution. To the neutralized solution, based on the total weight of acrylic acid, 2000 ppm of polyethylene glycol diacrylate (PEGDA) as an internal crosslinking agent, and 80 ppm of bis(2,4,6-trimethylbenzoyl)-phenylphosphine oxide (Irgacure^{®} 819) and 2000 ppm of sodium persulfate (SPS) as initiators were added to prepare a monomer composition.

The monomer composition was put in a rectangular reactor of 30cm x 30cm, and irradiated by UV at the intensity of 10mW/cm² to progress a polymerization reaction for 60 seconds, thus obtaining hydrogel polymer.

### (Grinding step)

The hydrogel polymer prepared in the step 1 was cut to a size of 5cm x 5cm, and the hydrogel was ground using a meat chopper, which is screw type cutting machine. Wherein, in the screw type cutting machine, a porous plate equipped with multiple cutting holes with a hole size of 16mm was used.

### (Drying step)

Thereafter, 1,000g of the ground super absorbent polymer hydrogel was put in an air vented dryer equipped with a porous plate. While maintaining the internal temperature of the dryer at 180°C, drying was conducted for 40 minutes to obtain resin powders. The powders were passed through a two-stage roll mill to obtain base resin (BR) powders.

### (Grinding and classifying step)

The base resin was ground to particles having particle diameters of 150µm to 850µm using a two-stage roll mill (GRAN-U-LIZERTM, MPE).

From the ground products, using a classifying sieve, base resin particles having particle diameters of 150µm to 850µm were selectively recovered.

### (Surface crosslinking step)

To 100g of the base resin particles obtained above, a surface crosslinking solution prepared by mixing 5g of water, 5.3g of propylene glycol, 0.1g of ethylene glycol diglycidyl ether, and 0.87g of 23 wt% aluminum sulfate aqueous solution was added and mixed for 2 minutes, and the mixture was subjected to a surface crosslinking reaction at 130°C for 50 minutes to prepare the final super absorbent polymer.

### Example 1

### (Polymer preparation step)

In a 10L glass container equipped with an agitator and thermometer, 1500g of acrylic acid, 6.3g of pentaerythritol triallyl ether as an internal crosslinking agent, and 3387g of water were agitated and mixed, while maintaining 5°C. Into the glass container containing the mixture, nitrogen was inflowed at 1000cc/min for 1 hour to substitute with a nitrogen condition. Next, 20.1g of 0.3% hydrogen peroxide aqueous solution, 22.5g of 1% ascorbic acid aqueous solution, and 45.0g of 2% 2,2'-azobis-(2-amidinopropane) dihydrochloride aqueous solution were added as polymerization initiators, and simultaneously, 22.3g of 0.01% ferrous sulfate was added as reducing agent to initiate polymerization. After the temperature of the mixture reached 85°C, polymerization was progressed at 90±2 °C for about 3 hours to obtain polymer.

### (Neutralization and grinding step)

While rotating a micronizer (F200, Karl Schnell) equipped with a porous plate including multiple holes having a hole size of 10 mm at 1500 rpm, 5000g of the obtained polymer was put therein and ground. Wherein, an aqueous solution of 1.5 wt% glycerol monolaurate (GML) was added such that the concentration of GML became 1,000 ppm.

Thereafter, while rotating a screw type cutting machine, meat chopper, equipped with a porous plate including multiple holes having a hole size of 6 mm at 500 rpm, the ground polymer was put therein to prepare secondary aggregated particles, which process was repeated three times. Wherein, in the first round, 220g of 50 wt% NaOH aqueous solution was added per 1000g of polymer. In the second round, 39.3g of 10 wt% Na₂SO₄ aqueous solution was added per 1000g of polymer after the first round. In the third round, the polymer was passed through without adding additives, to prepare hydrated super absorbent polymer particles.

### (Drying step)

1,000g of the hydrated super absorbent polymer particles were dried with a 120°C belt dryer for 40 minute to obtain rein powders. The powders were passed through a two-stage roll mill to obtain base resin powders.

### (Surface crosslinking step)

Next, to 100g of the obtained base resin powders, a surface crosslinking solution prepared by mixing 4g of water, 6g of methanol, 0.30g of ethylene gylcol diglycidyl ether (EJ-1030S), 0.1g of propylene glycol and 0.2g of aluminum sulfate was added and mixed for 1 minute, and the mixture was subjected to a surface crosslinking reaction at 140°C for 50 minutes, thus obtaining surface-crosslinked super absorbent polymer.

### Example 2

Super absorbent polymer was prepared by the same method as Example 1, except that in the neutralization and grinding step, an aqueous solution of 1.5 wt% GML was added such that the concentration of GML became 5,000 ppm, based on acrylic acid.

### Comparative Example 2

Super absorbent polymer was prepared by the same method as Example 1, except that in the neutralization and grinding step, an aqueous solution of 1.5 wt% GML was added such that the concentration of GML became 20,000 ppm, based on acrylic acid.

### Experimental Example 1: Property measurement of super absorbent polymer

Using the property measuring apparatus (100) of super absorbent polymer as shown in Fig. 1, the properties of each super absorbent polymer of Examples and Comparative Examples were measured as follows. The measurement temperature was maintained at 24°C.

On a scale(13, AND electronic balance GF-3000) connected with a computer for collecting data, a first water tank(10) was placed, and a pipe(11) was installed to penetrate through the upper cover of the first water tank, so that the internal pressure of the first water tank remains the same as atmospheric pressure. As the first water tank, a cylindrical acryl water tank having an internal diameter of 10 cm, an internal height of 30 cm, and an internal volume of 2355 cm³ was used, and as the pipe, a glass pipe having a diameter of 1 cm, and a height of 30 cm was used.

Separately, as the second water tank(20), an acryl water tank having an internal width and length of 20 cm, respectively, an internal height of 5 cm, and an internal volume of 2000 cm³, and equipped with a glass filter(21, Chemglass CG-201-52, diameter 80 mm, thickness 6.35 mm, extra coarse) on the center of the upper cover was prepared.

The second water tank was placed next to the scale, and a connection pipe(14) was installed between the lower part of the first water tank (about 1/5 point of the height of the water tank) and the lower part of the second water tank (about 1/5 point of the height of the water tank). As the connection pipe, a pipe made of silicone, and having a diameter of 10 mm and a length of 50 cm was used.

Next, the first water tank was filled with physiological saline (0.9 wt%, sodium chloride aqueous solution), so that the second water tank was entirely filled with physiological saline. When the glass filter of the second water tank was saturated with physiological saline, the initial weight of the first water tank was measured.

Separately, a cylinder(22) made of polymethylmethacrylate, having an internal diameter of 60mm and a height of 50mm, equipped with a stainless mesh of 400 mesh (Material No. 1.4401 / AISI 316 mesh 400, hole size 36 µm, thread diameter 28 µm), and having an open upper surface was prepared. Through the upper surface of the cylinder, 1g of super absorbent polymer particles(23, particle diameter 150 to 850µm) were added, and the particles were uniformly spread on the mesh without overlapping each other, and then, immediately after the glass filter of the second water tank was saturated with physiological saline, the cylinder was laid thereon such that the mesh touched the glass filter.

From immediately after the cylinder was laid on the glass filter, the weight of the first tank was continuously collected using a computer. Namely, the amount(C) of water absorbed by super absorbent polymer was measured from the reduced weight of the first water tank, and the absorption speed(V) of super absorbent polymer was derived from the amount of water absorbed by super absorbent polymer per second. The data were collected until the super absorbent polymer was saturated and the weight of the first water tank was not reduced any longer, thus deriving the maximum value of absorption speed(Vmax), a time when the maximum value of absorption speed(Tmax) is achieved, and the amount of physiological saline absorbed in super absorbent polymer for 5 minutes.

The measurement results are described in the following Table 1. In the case of Comparative Example 2, the absorption speed repeated rise and drop without exceeding 0.1 g/g.sec, and thus, Vma and Tmax could not be derived.

**[Table 1]**

| Properties | Comparative Example 1 | Comparative Example 2 | Example 1 | Example 2 |
|---|---|---|---|---|
| Vmax (g/g·sec) | 0.34 | Non measurable | 0.52 | 0.48 |
| Tmax (sec) | 5 | Non measurable | 10 | 15 |
| C5min (g) | 31.6 | 16.7 | 45.0 | 45.8 |

### Experimental Example 2: Preparation and evaluation of absorption core

Using each super absorbent polymer prepared in Example 1 and Comparative Example 1, absorption cores were prepared as follows, and the absorption speeds and rewet properties were measured.

### (1) Preparation of absorption core

2 pieces of air-laid paper (HungChin, 37 gsm) and 1 piece of TABCW non-woven fabric (Jinrou, 38 gsm) were respectively cut to a size of width 380 mm x length 110 mm. On the air-laid non-oven fabric, 5.8 g of super absorbent polymer powders (particle diameter 150 to 850µm) were spread, and then, the TABCW non-woven fabric was laminated, 5.8 g of super absorbent polymer powders were spread again, and then, the air-laid non-woven fabric was laminated. Each layer was adhered with hot melt adhesive (total 8 gsm used) to prepare an absorption core.

### (2) Measurement of absorption speed

In the absorption core, 80 ml of physiological saline (0.9 wt%, sodium chloride aqueous solution) was injected two times at 5-minute intervals, and at each injection, a time (absorption speed, seconds) taken until the physiological saline completely disappeared on the core surface was measured.

### (3) Measurement of rewet

20 pieces of filter paper (whatman company, no. 4, 20-25 µm, diameter 110 mm) were prepared, and the weight was measured.

1 hour after the measurement of the physiological saline absorption speed (2) was completed, 20 pieces of filter paper were put on the absorption core, and a pressure of 0.7 psi was applied for 1 minute. Thereafter, the weight of the filter paper was measured, and a difference from the initial weight was calculated to measure the amount(g) of physiological saline that permeates in the filter paper.

**[Table 2]**

| Properties | Comparative Example 1 | Example 1 |
|---|---|---|
| 1^{st} absorption speed (s) | 17 | 12 |
| 2^{nd} absorption speed (s) | 20 | 13 |
| Rewet (g) | 3.0 | 1.0 |

From the results, it could be confirmed that the super absorbent polymer of Example 1 fulfilling that the maximum value of absorption speed (Vmax) is greater than 0.4 g/g sec, a time when the maximum value of absorption speed is achieved, is greater than 5 second to less than 120 seconds, and the amount of physiological saline absorbed in super absorbent polymer for 5 minutes (C5min) is greater than 40g, compared to the super absorbent polymer of Comparative Example 1 failing to fulfilling the above requirements, not only exhibit excellent absorption speed, but also has remarkably improved rewet prevention property.

### [Reference Numerals]

10: first water tank
11: pipe
12: physiological saline
13: scale
14: connection pipe
20: second water tank
21: glass filter
22: cylinder
23: super absorbent polymer
100: property measuring apparatus of super absorbent polymer

## Claims

1. Super absorbent polymer wherein
when the super absorbent polymer is spread on the surface of a porous substrate to which physiological saline (0.9 wt%, NaCl aqueous solution) is continuously fed, and the amount of physiological saline that the super absorbent polymer absorbs from the surface of the porous substrate, and the absorption speed are measured continuously from the time the super absorbent polymer is spread,
the maximum value of the absorption speed (Vmax) is greater than 0.4 g/g·sec,
a time when the maximum value of the absorption speed (Tmax) is achieved, is greater than 5 seconds to less than 120 seconds, and
the amount of physiological saline absorbed in the super absorbent polymer for 5 minutes (C5min) is greater than 40 g.

2. The super absorbent polymer according to claim 1, wherein the maximum value of the absorption speed (Vmax) is 0.45 g/g sec to 1 g/g sec.

3. The super absorbent polymer according to claim 1 or 2, wherein the time when the maximum value of the absorption speed (Tmax)is achieved, is 8 seconds to 40 seconds.

4. The super absorbent polymer according to any one of claims 1 to 3, wherein the amount of physiological saline absorbed in the super absorbent polymer for 5 minutes (C5min) is 45 g to 55 g.
